# EUROPEAN PATENT APPLICATION

(11) **EP 4 847 445 A1**
(43) Date of publication of application: **02.12.2026**
(21) Application number: 25179564.7
(22) Date of filing: 28.05.2025
(51) Int. Cl.: C01B 3/38, C01C 1/04, C07C 273/04

(54) **SYSTEM AND PROCESS FOR PRODUCING FEED FOR AMMONIA AND UREA PRODUCTION**

(71) Applicant: Sabic Agri-Nutrients Company, 31961 Jubail (SA)
(72) Inventor: SIVASUBRAMANIAN, Ashok, 562125 Karnataka (IN); KUMAR, Arvind, 23955 Thuwal (SA); GUNASEKARAN, Sivalingam, 6160 GA (NL)
(74) Representative: Sabic Intellectual Property Group

(57) **Abstract**

Disclosed is a process for reducing carbon dioxide in production of ammonia and urea. The process can include producing a first reformed gas stream (205) by reacting hydrocarbons in a first stream with steam in a primary reformer (210), producing a nitrogen gas enriched stream (207) by contacting the first reformed gas stream and air in a secondary reformer (220), producing a second reformed gas stream (209) by reacting hydrocarbons in the nitrogen gas enriched stream (207) and hydrocarbons in a second stream with steam in a gas heated reformer (230), reducing heat of the second reformed gas stream and producing a first boiler produced steam (262), compressing carbon dioxide to produce a low pressure compressed carbon dioxide by powering a steam turbine carbon dioxide compressor by the first boiler produced steam, and compressing low pressure compressed carbon dioxide to produce a high pressure compressed carbon dioxide by an electric motor driven high pressure compressor.

## Description

### CROSS-REFERENCE TO RELATED APPLICATIONS

This application claims priority to India Application No. 202541021582, filed March 11, 2025, the contents of which is incorporated into the present application by reference.

### FIELD OF THE DISCLOSURE

This disclosure generally relates to system and process for capturing and/or reducing carbon dioxide. The process can reduce the amount of carbon dioxide produced in the production of ammonia and urea.

### DESCRIPTION OF RELATED ART

Carbon capture and/or storage is in growing demand worldwide as companies, industries, and countries are looking for ways to meet increasingly demanding regulations and environmental goals. Conventional Carbon Capture and Storage (CCS) technologies are not likely to meet these demands and goals. CCS technologies are faced with drawbacks such as high energy consumption, transportation difficulties, difficulties in meeting need of immediate utilization of captured carbon, and leakage of captured carbon to the atmosphere during storage.

Urea is widely used in agricultural industry as a nitrogen fertilizer. Urea is cheap, has a high nitrogen content, and has a low transport and storage cost. The current global urea market size is valued at more than 100 billions, and is projected to grow. However, current methods of producing urea are carbon intensive.

In urea production plants compressed carbon dioxide is reacted with ammonia to produce urea. Carbon dioxide is compressed using steam driven compressors. The steam consumed for the carbon dioxide compressing may be supplied by offsite boilers and ammonia production plant's excess steam exports. These offsite boilers are typically powered by burning natural fuel gas that produces carbon dioxide. Burning natural gas produces flue gases that contain in some instances 6 - 12 vol% CO₂ that is, in some instances, vented to the atmosphere. There is a need to capture or reduce the CO₂ that is emitted to the atmosphere from the flue gas.

### SUMMARY

A discovery has been made that provides a solution to at least one or more of the aforementioned problems associated with urea production. In one aspect, a process for reducing steam required for urea production is provided. The process includes compressing a portion of carbon dioxide used for urea production using an electric driven carbon dioxide compressor. Use of electric driven carbon dioxide compressor can reduce the amount of steam used for carbon dioxide compression, which can reduce the load on the offsite boilers, which in turn can reduce fuel gas usage, making the process relatively more carbon efficient. However, as the load on the offsite boilers are reduced, to run the boilers with good efficiency and operating load, steam supplied to the boilers by the ammonia production plants needs to be reduced. The process reduces the steam supplied to the offsite boilers by reducing the amount of steam produced by the ammonia production plants. The amount of steam produced by the ammonia production plants is reduced by using a gas heated reformer downstream of a secondary reformer used in the ammonia production plants.

Some aspects of the disclosure are directed to a process for reducing the amount of carbon dioxide produced in production of ammonia and urea. The process can include any one of, any combination of, or all of steps (i) to (vi). In step (i), a first reformed gas stream can be produced by reacting at least a portion of hydrocarbons in a first stream with steam in a primary reformer. The first reformed gas stream can contain products of the reaction and unreacted hydrocarbons from the first stream. In step (ii), a nitrogen gas enriched stream can be produced by reacting at least a portion of hydrocarbons in the first reformed gas stream with air in a secondary reformer. The first reformed gas stream can be obtained from the primary reformer. The nitrogen gas enriched stream can contain products of the reaction in the secondary reformer, nitrogen (N₂) from the air, and unreacted hydrocarbons from the first reformed gas stream. In step (iii), a second reformed gas stream can be produced by reacting at least a portion of hydrocarbons in the nitrogen gas enriched stream, and at least a portion of hydrocarbons in a second stream with steam in a gas heated reformer. The nitrogen gas enriched stream can be obtained from the secondary reformer. The second reformed gas stream can contain products of the reaction in the second reformer, unreacted hydrocarbons and nitrogen from the nitrogen gas enriched stream, and unreacted hydrocarbons from the second stream. Step (iv) can include reducing heat of the second reformed gas stream and producing a first boiler produced steam and a cooled second reformed gas stream by heating water and/or steam in a first boiler with heat from the second reformed gas stream. In step (v), a low pressure compressed carbon dioxide can be produced by compressing carbon dioxide by a steam turbine carbon dioxide compressor. The steam turbine carbon dioxide compressor can be powered by the first boiler produced steam. In step (vi), a high pressure compressed carbon dioxide can be produced by compressing the low pressure compressed carbon dioxide by a high pressure electric compressor. The low pressure compressed carbon dioxide can be obtained from the steam turbine carbon dioxide compressor. The high pressure electric compressor can be powered by an electric motor. In certain embodiments, the electric motor is powered by electricity from a renewable energy source. The renewable energy source can include but is not limited to wind energy source, solar energy source, bioenergy source, hydroelectric energy source, and/or hydrothermal energy source. The cooled second reformed gas stream can be used as a feed for producing ammonia. The high pressure compressed carbon dioxide can be used as a feed for producing urea.

The process can produce less carbon dioxide than a reference process that does not use the gas heated reformer, or does not use the electric motor driven high pressure electric compressor, or does not use both. The process can use less steam to produce the high pressure compressed carbon dioxide than a reference process that does not use the electric motor driven high pressure electric compressor. The reference process can use the primary reformer, the secondary reformer, the first boiler, the steam turbine carbon dioxide compressor, and a high pressure compressor driven by steam.

The second reformed gas stream produced in the gas heated reformer can have a temperature greater than the cooled second reformed gas stream produced in the first boiler. In certain embodiments, the second reformed gas stream produced in the gas heated reformer has a temperature of 700 to 900 °C, such as a temperature of, greater than, less than, or between 700 °C, 720 °C, 740 °C, 760 °C, 780 °C, 800 °C, 820 °C, 840 °C, 860 °C, 880 °C, or 900 °C, or range thereof. In certain embodiments, the cooled second reformed gas stream produced in the first boiler has a temperature of 250 to 500 °C, such as a temperature of, greater than, less than, or between 250 °C, 260 °C, 280 °C, 300 °C, 320 °C, 340 °C, 360 °C, 380 °C, 400 °C, 420 °C, 440 °C, 460 °C, 480 °C, or 500 °C, or range thereof. The nitrogen gas enriched stream can have a temperature greater than the second reformed gas stream produced in the gas heated reformer. In certain embodiments, the nitrogen gas enriched stream has a temperature of 900 °C to 1200 °C, such as a temperature of, greater than, less than, or between 900 °C, 920 °C, 940 °C, 960 °C, 980 °C, 1000 °C, 1020 °C, 1040 °C, 1060 °C, 1080 °C, 1100 °C, 1120 °C, 1140 °C, 1160 °C, 1180 °C, or 1200 °C, or range thereof. In certain embodiments, the first reformed gas stream produced in the primary reformer has a temperature of 700 to 900 °C, such as a temperature of, greater than, less than, or between 700 °C, 720 °C, 740 °C, 760 °C, 780 °C, 800 °C, 820 °C, 840 °C, 860 °C, 880 °C, or 900 °C, or range thereof. The first boiler produced steam can have a temperature greater than that of the water and/or steam that is heated in the first boiler.

In certain embodiments, water is separated from the first boiler produced steam in a steam drum to produce a dry steam, and the dry steam is used to power the steam turbine carbon dioxide compressor. In certain embodiments, the water separated from the first boiler produced steam in the steam drum is recycled to the first boiler. In certain embodiments, the water recycled to the first boiler from the steam drum forms at least a portion of the water that is heated in the first boiler to produce the first boiler produced steam. In certain embodiments, the dry steam is passed through a third boiler upstream of providing the dry steam to the steam turbine carbon dioxide compressor. The third boiler can control the amount of dry steam provided to the steam turbine carbon dioxide compressor to power it. The third boiler can be an outside battery limit (OSBL) boiler.

In certain embodiments, the steam reacted in the primary reformer, and/or the steam reacted in the gas heated reformer is supplied at least in part by a second boiler. In certain embodiments, the steam reacted in the primary reformer, and/or the steam reacted in the gas heated reformer is not supplied by a second boiler.

The first stream can contain hydrocarbons and steam. In certain embodiments, the first stream has a steam to carbon mole ratio of 2.7:1 to 4:1. In certain embodiments, the first stream at a dry basis contains 78 to 98 mol. % of methane, 0 to 12 mol. % of ethane, 0 to 8 mol. % of propane, 0 to 4 mol. % of iso-butane, 0 to 4 mol. % of n-butane, and/or 0 to 2 mol. % of C5+ hydrocarbons. In certain embodiments, the at least a portion of the hydrocarbons in the first stream and steam can be reacted in the primary reformer at a pressure of 28 to 50 barg, and/or temperature of 680 to 840 °C, in presence of a catalyst, such as a nickel based catalyst. In certain embodiments, the first reformed gas stream contains at a dry basis, 8 to 13 mol. % of CO₂, 4 to 12 mol. % of CO, 50 to 70 mol. % of H₂, and/or 7 to 30 mol. % of methane.

In certain embodiments, at least a portion of the hydrocarbons in the first reformed gas stream and air can be reacted in the secondary reformer at a pressure of 27 to 49 barg, and/or a temperature of 900 to 1150 °C, in presence of a catalyst, such as a nickel based catalyst. In certain embodiments, the nitrogen gas enriched stream at a dry basis contains 6 to 10 mol. % of CO₂, 8 to 14 mol. % of CO, 45 to 58 mol. % of H₂, 20 to 30 mol. % of N₂, and/or 0.2 to 1.8 mol. % of methane.

The second stream can contain hydrocarbons and steam. In certain embodiments, the second stream has a steam to carbon mole ratio of 2.7 to 4. In certain embodiments, the second stream at a dry basis contains 78 to 98 mol. % of methane, 0 to 12 mol. % of ethane, 0 to 8 mol. % of propane, 0 to 4 mol. % of iso-butane, 0 to 4 mol. % of n-butane, and/or 0 to 2 mol % of C5+ hydrocarbons. At least a portion of the hydrocarbons in the second stream and at least a portion of the hydrocarbons in the nitrogen gas enriched stream can be reacted with steam in the gas heated reformer at a pressure of 27 to 49 barg and/or a temperature of 750 to 850 °C, in presence of catalyst, such as a nickel based catalyst. In certain embodiments, the second reformed gas stream contains at a dry basis contains 6 to 10 mol. % of CO₂, 8 to 14 mol. % of CO, 45 to 58 mol. % of H₂, 20 to 30 mol. % of N₂, and/or 0.2 to 2 mol. % of methane. In certain embodiments, the first stream and the second stream is at least in part supplied by a same hydrocarbon stream. In certain embodiments, the first stream can be formed by contacting the hydrocarbon stream with steam. In certain embodiments, the second stream can be formed by contacting the hydrocarbon stream with steam.

The high pressure compressed carbon dioxide compressed by the high pressure electric compressor can have a pressure of 10 to 35 megapascals, such as a pressure of, greater than, less than, or between 10, 11, 12, 13, 14, 15, 16, 17, 18, 19, 20, 21, 22, 23, 24, 25, 26, 27, 28, 29, 30, 31, 32, 33, 34, or 35 megapascals, or range thereof. The low pressure carbon dioxide compressed by the steam turbine carbon dioxide compressor can have a pressure of 1 to 5 megapascals, such as a pressure of, greater than, less than, or between 1, 2, 3, 4, or 5 megapascals, or range thereof. In certain embodiments, the steam turbine carbon dioxide compressor and the high pressure electric compressor are not coupled by a gear box connected to the steam turbine carbon dioxide compressor. In certain embodiments, the high pressure electric compressor is not powered by steam, such as the dry steam.

In certain embodiments, the process further includes reacting the high pressure compressed carbon dioxide with ammonia to produce urea. In certain embodiments, the process further includes producing ammonia by reacting nitrogen from the cooled second reformed gas stream from the first boiler. In certain embodiments, the process further includes producing ammonia by reacting hydrogen from the first reformed gas stream, the second reformed gas stream, and/or the cooled second reformed gas stream. In certain embodiments, at least a portion of the ammonia used to produce urea is formed by reacting the nitrogen from the cooled second reformed gas stream from the first boiler.

Certain aspects are directed to a system for production of nitrogen gas and compressed carbon dioxide. The system can be used to perform the process for reducing the amount of carbon dioxide produced in production of ammonia and urea, as described herein. The system can include the primary reformer, the secondary reformer, the gas heated reformer, the first boiler, the steam turbine carbon dioxide compressor, the high pressure electric compressor, and the electric motor. The primary reformer can contain a steam and/or first stream inlet, and a first reformed gas stream outlet. The secondary reformer can contain a first reformed gas stream inlet in fluid communication with the first reformed gas stream outlet of the primary reformer, the secondary reformer also having an air inlet and a nitrogen gas enriched stream outlet. The gas heated reformer can contain a nitrogen gas enriched stream inlet in fluid communication with the nitrogen gas enriched stream outlet of the secondary reformer, the gas heated reformer also having a steam and/or second stream inlet, and a second reformed gas stream outlet. The first boiler can contain a second reformed gas stream inlet in fluid communication with the second reformed gas stream outlet of the gas heated reformer, the first boiler also having a water and/or steam inlet, a first boiler produced steam outlet, and a cooled second reformed gas stream outlet. The steam turbine carbon dioxide compressor can contain a first boiler produced steam inlet in fluid communication with the first boiler produced steam outlet of the first boiler, the steam turbine carbon dioxide compressor also having a carbon dioxide inlet and a low pressure compressed carbon dioxide outlet. The high pressure electric compressor can contain a low pressure compressed carbon dioxide inlet in fluid communication with the low pressure compressed carbon dioxide outlet of the steam turbine carbon dioxide compressor, the high pressure electric compressor also having a high pressure compressed carbon dioxide outlet. The electric motor can be in mechanical communication with the high pressure electric compressor.

In certain embodiments, the system contains the second boiler. The second boiler can contain a second boiler steam outlet in fluid communication with i) the steam and/or first stream inlet of the primary reformer, ii) the steam and/or second stream inlet of the gas heated reformer, or both. In certain embodiments, the system further contains a renewable energy source in electrical communication with the electric motor. The renewable energy source can include but is not limited to wind energy source, solar energy source, bioenergy source, hydroelectric energy source, and/or hydrothermal energy source.

The first stream can be fed to the primary reformer through the steam and/or first stream inlet. The primary reformer can be configured to react at least a portion of the hydrocarbons in the first stream with steam to produce the first reformed gas stream. The first reformed gas stream can exit the primary reformer through the first reformed gas stream outlet.

The first reformed gas stream can be fed to the secondary reformer through the first reformed gas stream inlet. Air can be fed to the secondary reformer through the air inlet. The secondary reformer can be configured to react at least a portion of the hydrocarbons in the first reformed gas stream with air to produce the nitrogen gas enriched stream. The nitrogen gas enriched stream can exit the secondary reformer through the nitrogen gas enriched stream outlet.

The nitrogen gas enriched stream can be fed to the gas heated reformer through the nitrogen gas enriched stream inlet. The second stream can be fed to the gas heated reformer through the steam and/or second stream inlet. The gas heated reformer can be configured to react at least a portion of the hydrocarbons in the nitrogen gas enriched stream and at least a portion of the hydrocarbons in the second stream with steam to produce the second reformed gas stream. The second reformed gas stream can exit the gas heated reformer through the second reformed gas stream outlet.

The second reformed gas stream can be fed to the first boiler through the second reformed gas stream inlet. Water and/or steam can be fed to the first boiler through the water and/or steam inlet. The first boiler can be configured to heat the water and/or steam fed to the first boiler with heat from the second reformed gas stream to produce the first boiler produced steam and the cooled second reformed gas stream. The cooled second reformed gas stream can exit the first boiler through the cooled second reformed gas stream outlet. The first boiler produced steam can exit the first boiler through the first boiler produced steam outlet.

Carbon dioxide can be fed to the steam turbine carbon dioxide compressor through the carbon dioxide inlet. The first boiler produced steam can be fed to the steam turbine carbon dioxide compressor through the first boiler produced steam inlet. The steam turbine carbon dioxide compressor can be configured to compress the carbon dioxide fed to the steam turbine carbon dioxide compressor to produce the low pressure compressed carbon dioxide. The low pressure compressed carbon dioxide can exit the steam turbine carbon dioxide compressor through the low pressure compressed carbon dioxide outlet. The steam turbine carbon dioxide compressor can be configured to be powered by the first boiler produced steam.

The low pressure compressed carbon dioxide can be fed to the high pressure electric compressor through the low pressure compressed carbon dioxide inlet. The high pressure electric compressor can be configured to compress the low pressure carbon dioxide and produce the high pressure compressed carbon dioxide. The high pressure compressed carbon dioxide can exit the high pressure electric compressor through the high pressure compressed carbon dioxide outlet. The electric motor can be configured to power the high pressure electric compressor.

In certain embodiments, the system contains an ammonia reactor. The ammonia reactor can contain a cooled second reformed gas stream inlet in fluid communication with the cooled second reformed gas stream outlet of the first boiler, and an ammonia outlet. The cooled second reformed gas stream from the first boiler can be fed to the ammonia reactor through the cooled second reformed gas stream inlet. The ammonia reactor can be configured to react the nitrogen and/or hydrogen (H₂) in the cooled second reformed gas stream to produce ammonia. The ammonia produced can exit the ammonia reactor through the ammonia outlet.

In certain embodiments, the system contains a urea reactor. The urea reactor can contain a high pressure compressed carbon dioxide inlet, an ammonia inlet, and a urea outlet. The high pressure compressed carbon dioxide inlet can be in fluid communication with the high pressure compressed carbon dioxide outlet of the high pressure electric compressor. The ammonia inlet of the urea reactor can be in fluid communication with the ammonia outlet of the ammonia reactor. The urea reactor can be configured to react the high pressure compressed carbon dioxide and ammonia to produce urea. The urea produced can exit the urea reactor through the urea outlet.

In certain embodiments, the system contains the steam drum. The steam drum can contain a water and/or steam outlet, a first boiler produced steam inlet, and a dry steam outlet. The water and/or steam outlet can be in fluid communication with the water and/or steam inlet of the first boiler. The first boiler produced steam inlet of the steam drum can be in fluid communication with the first boiler produced steam outlet of the first boiler. The first boiler produced steam from the first boiler can be fed to the steam drum through the first boiler produced steam inlet of the steam drum. The steam drum can be configured to separate water from the first boiler produced steam to produce the dry steam. The dry steam can exit the steam drum through the dry steam outlet. The water separated can exit the steam drum through the water/stream outlet and can be fed to the first boiler through the water and/or steam inlet of the first boiler.

In certain embodiments, the system contains the third boiler. The third boiler can contain a dry steam inlet and a dry steam outlet. The dry steam inlet of the third boiler can be in fluid communication with the dry steam outlet of the steam drum. The dry steam outlet of the third boiler can be in fluid communication with the first boiler produced steam inlet of the steam turbine carbon dioxide compressor. Dry steam from the steam drum can be fed to the third boiler through the dry steam inlet of the third boiler. Dry steam can exit the third boiler through the dry steam outlet of the third boiler. The third boiler can be configured to control the amount of steam that is fed to the steam turbine carbon dioxide compressor. In certain embodiments, when the system contains the steam drum and third boiler, instead of feeding the first boiler produced steam directly to the steam turbine carbon dioxide compressor, the dry steam formed from the third boiler produced steam may be fed to the steam turbine carbon dioxide compressor.

The first stream, first reformed gas stream, nitrogen gas enriched stream, second stream, second reformed gas stream, cooled second reformed gas stream, and first boiler produced steam can have chemical composition and physical properties as described herein, such as for the process for reducing the amount of carbon dioxide produced in production of ammonia and urea.

The following includes definitions of various terms and phrases used throughout this specification.

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

The terms "wt.%," "vol.%," or "mol.%" refers to a weight percentage of a component, a volume percentage of a component, or molar percentage of a component, respectively, based on the total weight, the total volume of material, or total moles, that includes the component. In a non-limiting example, 10 grams of component in 100 grams of the material is 10 wt.% of component. The "wt.%," "vol.%," or "mol.%" of a component in a stream at a dry basis refers to the weight percentage, volume percentage, or molar percentage of the component in the stream respectively, based on the total weight, the total volume, or total moles of material excluding the weight, volume, or moles of water, respectively, in the stream. In a non-limiting example, 10 moles of component in a stream containing a total of 110 moles of materials with 10 moles of water, is 10 mol. % of component in the stream at a dry basis.

The term "ppm" refer to parts per million by weight, based on the total weight, of material that includes the component.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the words "a" or "an" when used in conjunction with any of the terms "comprising," "including," "containing," or "having" in the claims, or the specification, may mean "one," but it is also consistent with the meaning of "one or more," "at least one," and "one or more than one."

The phrase "and/or" means and or or. To illustrate, A, B, and/or C includes: A alone, B alone, C alone, a combination of A and B, a combination of A and C, a combination of B and C, or a combination of A, B, and C. In other words, "and/or" operates as an inclusive or.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or process steps.

The covering of the present invention can "comprise," "consist(s) essentially of," or "consist of" particular steps, components, compositions, *etc.* disclosed throughout the specification. In one aspect of the present invention, and with reference to the transitional phrase "consist(s) essentially of" or "consisting essentially of," a basic and novel characteristic of the present invention can include a process for reducing carbon dioxide in production of ammonia and urea. The process can include producing a first reformed gas stream by reacting hydrocarbons in a first stream with steam in a primary reformer, producing a nitrogen gas enriched stream by contacting the first reformed gas stream and air in a secondary reformer, producing a second reformed gas stream by reacting hydrocarbons in the nitrogen gas enriched stream and hydrocarbons in a second stream with steam in a gas heated reformer, reducing heat of the second reformed gas stream and producing a first boiler produced steam, compressing carbon dioxide to produce a low pressure compressed carbon dioxide by powering a steam turbine carbon dioxide compressor by the first boiler produced steam, and compressing low pressure compressed carbon dioxide to produce a high pressure compressed carbon dioxide by an electric motor driven high pressure compressor.

Other objects, features and advantages of the present invention will become apparent from the following detailed description and examples. It should be understood, however, that the detailed description and examples, while indicating specific embodiments of the invention, are given by way of illustration only and are not meant to be limiting. Additionally, it is contemplated that changes and modifications within the spirit and scope of the invention will become apparent to those skilled in the art from this detailed description. In further embodiments, features from specific embodiments or aspects may be combined with features from other embodiments and aspects. For example, features from one embodiment or aspect may be combined with features from any of the other embodiments or aspects. In further embodiments, additional features may be added to the specific embodiments described herein.

### BRIEF DESCRIPTION OF THE DRAWINGS

Advantages of the present invention may become apparent to those skilled in the art with the benefit of the following detailed description and upon reference to the accompanying drawings. While the invention is susceptible to various modifications and alternative forms, specific embodiments thereof are shown by way of example in the drawings. The drawings may not be to scale.
**FIG. 1****:** Schematic of a system for production of nitrogen (N₂) gas and compressed carbon dioxide according to an example of the present disclosure is shown.
**FIG. 2****:** Schematic of a nitrogen gas production unit of the system of FIG. 1 is shown.
**FIG. 3****:** Schematic of a compressed carbon dioxide production unit of the system of FIG. 1 is shown.
**FIG. 4****:** Schematic of a system for production of ammonia and urea according to an example of the present disclosure is shown
**FIG. 5****:** A flow chart of process steps employed for a process for producing nitrogen (N₂) gas and compressed carbon dioxide using the system of FIG. 1 is shown.
**FIG. 6****:** A flow chart of process steps employed for a process for producing urea and ammonia using the system of FIG. 4 is shown.

### DETAILED DESCRIPTION

A discovery has been made that provides a process for reducing the amount of carbon dioxide produced in production of ammonia and urea. The process can reduce steam required for producing compressed carbon dioxide used for urea production and thereby can reduce the load on the offsite boilers that supplies steam to the carbon dioxide compressors in a urea production plant. Reducing the load of the offsite boilers reduces the amount of fuel gas burnt to run the boilers, which in turn reduces the amount of carbon dioxide produced to run the boilers. Steam required for producing compressed carbon dioxide used for urea production is reduced by compressing a portion of the carbon dioxide using a electric powered compressor. The process also decreases the amount of steam produced in an ammonia production plant, thereby reducing the amount of steam supplied to the offsite boilers. Reducing the amount of steam supplied to the offsite boilers helps to run the boilers with good operating efficiency at a reduced load. The amount of steam produced in the ammonia production plant is reduced by using a gas heated reformer downstream of a secondary reformer used in the ammonia production plant.

These and other non-limiting aspects of the present disclosure are discussed in further detail in the following sections.

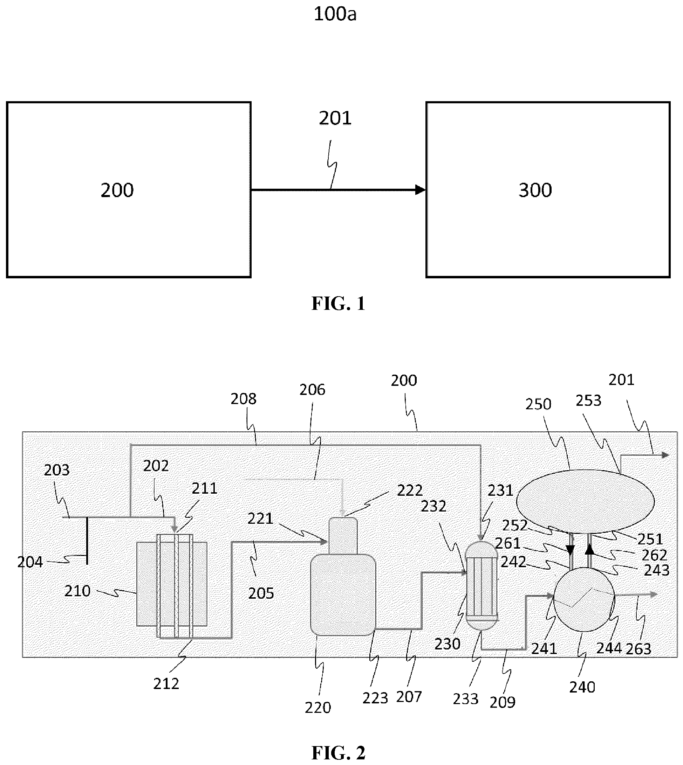

Referring to FIG. 1, a schematic of a system 100a, for production of nitrogen (N₂) gas and compressed carbon dioxide according to an example of the present disclosure is shown.

The system 100a can contain a nitrogen gas production unit 200 and a compressed carbon dioxide production unit 300. Steam from the unit 200 can be fed to the unit 300 via a steam stream 201.

Referring to FIG. 2, a schematic of the nitrogen gas production unit 200, of the system 100a is shown. The unit 200 can contain a primary reformer 210, a secondary reformer 220, a gas heated reformer 230, a first boiler 240 and a steam drum 250.

The primary reformer 210 can contain a first stream and/or steam inlet 211, a first reformed gas stream outlet 212. A first stream 202 containing hydrocarbons and steam can be fed to the primary reformer 210 through the first stream and/or steam inlet 211. The first stream 202 can be formed by contacting a hydrocarbon stream 203 with a steam feed stream 204. The hydrocarbon stream 203 can contain hydrocarbons. The steam feed stream 204 can contain steam. In the primary reformer 210, at least a portion of the hydrocarbons in the first stream can react with the steam. A first reformed gas stream 205 containing the products of the reaction of the at least a portion of the hydrocarbons in the first stream and steam, and unreacted hydrocarbons in the first stream, can be formed. The first reformed gas stream 205 formed can exit the primary reformer 210 through the first reformed gas stream outlet 212.

The secondary reformer 220 can contain a first reformed gas stream inlet 221, an air inlet 222, and a nitrogen gas enriched stream outlet 223. The first reformed gas inlet 221 can be in fluid communication with the first reformed gas stream outlet 212 of the primary reformer 210. The first reformed gas stream 205 can be fed to the secondary reformer 220 through the first reformed gas stream inlet 221. A air stream 206 containing air can be fed to the secondary reformer 220 through the air inlet 222. In the secondary reformer at least a portion of the hydrocarbons in the first reformed gas stream can be reacted with the air. A nitrogen gas enriched stream 207 containing the products of the reaction of the at least a portion of the hydrocarbons in the first reformed gas stream and air, unreacted hydrocarbons in the first reformed gas stream, and nitrogen (N₂) from the air, can be formed. The nitrogen gas enriched stream 207 formed can exit the secondary reformer 220 through the nitrogen gas enriched stream outlet 223.

The gas heated reformer 230 can contain a second stream and/or steam inlet 231, a nitrogen gas enriched stream inlet 232, and a second reformed gas stream outlet 233. The nitrogen gas enriched stream inlet 232 can be in fluid communication with the nitrogen gas enriched stream outlet 223 of the secondary reformer 220. A second stream 208 containing hydrocarbons and steam can be fed to the gas heated reformer 230 through the second stream and/or steam inlet 231. The second stream 208 can be formed by contacting the hydrocarbon stream 203 with the steam feed stream 204. The nitrogen gas enriched stream 207 can be fed to the gas heated reformer 230 through the nitrogen gas enriched stream inlet 232. In the gas heated reformer 230 at least a portion of the hydrocarbons in the second stream 208, and at least a portion of the hydrocarbons in the nitrogen gas enriched stream 207 can be reacted with steam. A second reformed gas stream 209 containing the products of the reaction of the at least a portion of the hydrocarbons in the second stream 208 and at least a portion of the hydrocarbons in the nitrogen gas enriched stream 207 with steam, unreacted hydrocarbons in the second stream, and unreacted hydrocarbons and nitrogen (N₂) from the nitrogen gas enriched stream, can be formed. The second reformed gas stream 209 formed can exit the gas heated reformer 230 through the second reformed gas stream outlet 233.

The first boiler 240 can contain a second reformed gas stream inlet 241, a water/steam inlet 242, a first boiler produced steam outlet 243, and a cooled second reformed gas outlet 244. The second reformed gas stream inlet 241 can be in fluid communication with the second reformed gas stream outlet 233 of the gas heated reformer 230. The second reformed gas stream 209 can be fed to the first boiler 240 through the second reformed gas stream inlet 241. A water/steam stream 261 containing water and/or steam can be fed to the first boiler through the water/steam inlet 242. In the first boiler 240 the water and/or steam fed via the stream 261 can be heated with heat from the second reformed gas stream 209 to produce a first boiler produced steam and a cooled second reformed gas stream. The cooled second reformed gas stream 263 can exit the first boiler 240 through the cooled second reformed gas stream outlet 244. The stream 262 containing the first boiler produced steam can exit the first boiler 240 through the first boiler produced steam outlet 243.

The steam drum 250 can contain a first boiler produced steam inlet 251, a water/steam outlet 252, and a dry steam outlet 253. The first boiler produced steam inlet 251 can be in fluid communication with the first boiler produced steam outlet 243 of the first boiler 240. The water/steam outlet 252 can be in fluid communication with the water/steam inlet 242 of the first boiler 240. The stream 262 can be fed to the steam drum 250 through the first boiler produced steam inlet 251. In the steam drum 250 water can be separated from the first boiler produced steam to produce dry steam. The stream 201 can contain the dry stream and can exit the steam drum 250 through the dry steam outlet 253. As discussed earlier, referring to FIG. 1, the stream 201 containing dry steam can be fed to the compressed carbon dioxide production unit 300. The stream 261 can contain the water separated from the first boiler produced steam. The stream 261 can exit the steam drum 250 via the water/steam outlet 252.

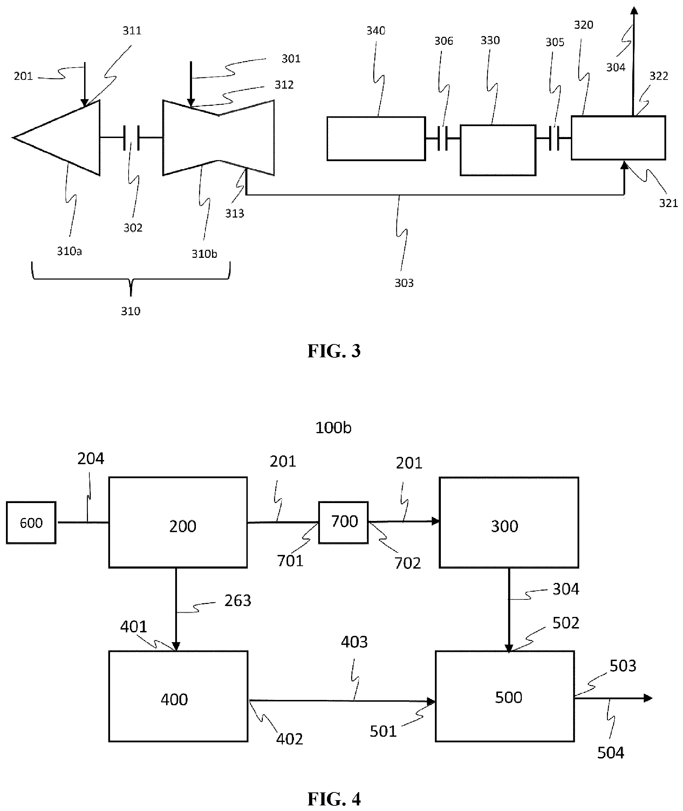

Referring to FIG. 3, a schematic of the compressed carbon dioxide production unit 300, of the system 100a is shown. The unit 300 can contain a steam turbine carbon dioxide compressor 310, a high pressure electric compressor 320, a electric motor 340 and a gear box 330.

The steam turbine carbon dioxide compressor 310 can contain a steam turbine 310a and a low pressure carbon dioxide compressor 310b. The steam turbine 310a can have a steam inlet 311 and can be mechanically connected to the low pressure carbon dioxide compressor 310b. The low pressure carbon dioxide compressor 310b can have a carbon dioxide inlet 312 and a low pressure compressed carbon dioxide outlet 313. The steam inlet 311 of the steam turbine 310a can be in fluid communication with the dry steam outlet 253 of the steam drum 250 of the unit 200, refer to FIG. 2.

Carbon dioxide can be fed to the low pressure carbon dioxide compressor 310b through the carbon dioxide inlet 312. The stream 201 containing dry steam from the steam drum 250 can be fed to the steam turbine 310a via the steam inlet 311. The dry steam can be used to run the steam turbine 310a to power the low pressure carbon dioxide compressor 310b using the mechanical connection 302. Powered by the steam turbine, the low pressure carbon dioxide compressor 310b can compress the input carbon dioxide 301 to form a low pressure compressed carbon dioxide. A stream 303 containing the low pressure compressed carbon dioxide can exit the low pressure carbon dioxide compressor 310b through the low pressure compressed carbon dioxide outlet 313.

The high pressure electric compressor 320 can contain a low pressure compressed carbon dioxide inlet 321 and a high pressure compressed carbon dioxide outlet 322. The low pressure compressed carbon dioxide inlet 321 can be in fluid communication with the low pressure compressed carbon dioxide outlet 313 of the low pressure carbon dioxide compressor 310b. The stream 303 can be fed to the high pressure electric compressor 320 through the low pressure compressed carbon dioxide inlet 321. The high pressure electric compressor 320 can compress the low pressure compressed carbon dioxide to form a high pressure compressed carbon dioxide. A high pressure compressed carbon dioxide stream 304 containing the high pressure compressed carbon dioxide can exit the high pressure electric compressor 320 through the high pressure compressed carbon dioxide outlet 322.

The gear box 330 can be mechanically connected with the high pressure electric compressor 320 via the connector 305. The electric motor 340 can be mechanically connected with the gear box 330 via the connector 306. The high pressure electric compressor 320 can be powered by the electric motor 340 via the gear box 330 and the connectors 305 and 306.

Referring to FIG. 4, a schematic of a system 100b, for production of ammonia and urea according to an example of the present disclosure is shown. The system 100b can contain a nitrogen gas production unit 200, a compressed carbon dioxide production unit 300, an ammonia production unit 400, a urea production unit 500, a second boiler 600, and a third boiler 700.

The units 200 and 300 of system 100b can be configured and operated similarly to the units 200 and 300 respectively of the system 100a. The steam feed stream 204, that is contacted with the hydrocarbon stream 203 to form the first stream 202 and the second stream 208, can be supplied by the second boiler 600. The stream 201 containing dry steam from the unit 200 can be passed through the third boiler 700 upstream of feeding it to the unit 300. The third boiler 700 can have a dry steam inlet 701 and dry steam outlet 702. The dry steam inlet 701 can be in fluid communication with the dry steam outlet 253 of the steam drum 250 of the unit 200. The dry steam outlet 702 can be in fluid communication with the steam inlet 311 of the steam turbine 310a of the unit 300.

The ammonia production unit 400 can have a second reformed gas stream inlet 401 and a ammonia outlet 402. The second reformed gas stream inlet 401 can be in fluid communication with the cooled second reformed gas stream outlet 244 of the first boiler 240 of the unit 200. The cooled second reformed gas stream 263 can be fed to the ammonia production unit 400 through the second reformed gas stream inlet 401. In the ammonia production unit 400 nitrogen (N₂) and/or hydrogen (H₂) contained in the cooled second reformed gas stream can be reacted to produce ammonia. An ammonia stream 403 containing the ammonia formed can exit the ammonia production unit 400 through the ammonia outlet 402. The ammonia production unit 400 can be any suitable system or unit capable of producing ammonia from nitrogen and hydrogen, known in the art. The nitrogen and hydrogen can be reacted to produce ammonia in the ammonia production unit 400 according to any suitable methods known in the art.

The urea production unit 500 can have a ammonia inlet 501, a compressed carbon dioxide inlet 502 and a urea outlet 503. The ammonia inlet 501 can be in fluid communication with the ammonia outlet 402 of the ammonia production unit 400. The compressed carbon dioxide inlet 502 can be in fluid communication with the high pressure compressed carbon dioxide outlet 322 of the high pressure electric compressor 320 of the unit 300. The ammonia stream 403 can be fed to the urea production unit 500 through the ammonia inlet 501. The high pressure compressed carbon dioxide stream 304 can be fed to the urea production unit 500 through the compressed carbon dioxide inlet 502. In the urea production unit 500, ammonia and the high pressure compressed carbon dioxide can be reacted to form urea. A urea stream 504 containing the urea formed can exit the urea production unit 500 through the urea outlet 503. The urea production unit 500 can be any suitable system or unit capable of producing urea from compressed carbon dioxide and ammonia, known in the art. The compressed carbon dioxide and ammonia can be reacted to produce urea in the urea production unit 500 according to any suitable methods known in the art.

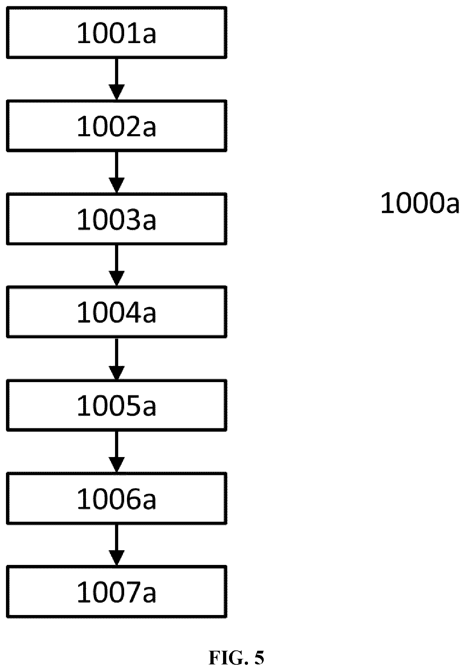

Referring to FIG. 5, a flow chart of process steps employed for a process 1000a for producing nitrogen (N₂) gas and compressed carbon dioxide using the system 100a is shown. The process 1000a can include steps 1001a, 1002a, 1003a, 1004a, 1005a, 1006a, and 1007a.

In step 1001a, the first reformed gas stream can be produced by reacting at least a portion of the hydrocarbons in the first stream with steam in the primary reformer. The step 1001a can be performed in the primary reformer 210 of the unit 200 of the system 100a, b. In step 1002a, the nitrogen gas enriched stream can be produced by reacting at least a portion of hydrocarbons in the first reformed gas stream with air in the secondary reformer. The step 1002a can be performed in the secondary reformer 220 of the unit 200 of the system 100a, b. In step 1003a, the second reformed gas stream can be produced by reacting at least a portion of the hydrocarbons in the nitrogen gas enriched stream and at least a portion of the hydrocarbons in the second stream with steam in the gas heated reformer. The step 1003a, can be performed in the gas heated reformer 230 of the unit 200 of the system 100a, b. Step 1004a can include reducing heat of the second reformed gas stream and producing the first boiler produced steam and the cooled second reformed gas stream by heating water and/or steam in the first boiler with heat from the second reformed gas stream. The step 1004a, can be performed in the first boiler 240 of the unit 200 of the system 100a, b. Step 1005a can include separating water from the first boiler produced steam to produce the dry steam. The step 1005a can be performed in the steam drum 250 of the unit 200 of the system 100a, b. In step 1006a, the low pressure compressed carbon dioxide can be produced by compressing carbon dioxide by the steam turbine carbon dioxide compressor. The step 1006a can be performed in the steam turbine carbon dioxide compressor 310 of the unit 300 of the system 100a, b. In step 1007a, the high pressure compressed carbon dioxide can be produced by compressing the low pressure compressed carbon dioxide by the high pressure electric compressor. The step 1007a, can be performed in the high pressure electric compressor 320 of the unit 300 of the system 100a, b.

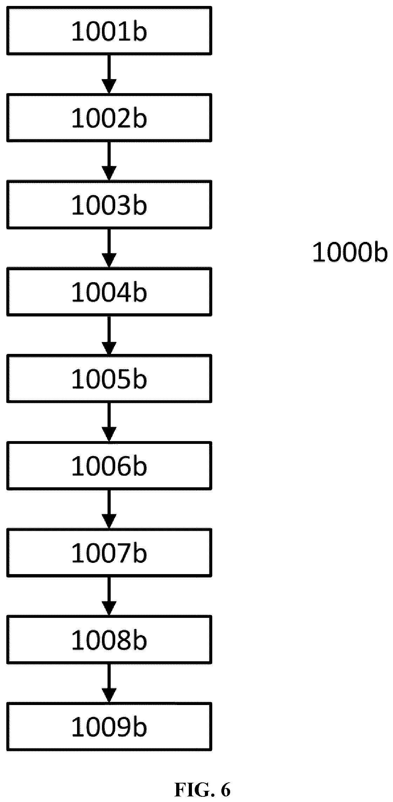

Referring to FIG. 6, a flow chart of process steps employed for a process 1000b for producing ammonia and urea using the system 100b is shown. The process can include steps 1001b, 1002b, 1003b, 1004b, 1005b, 1006b, 1007b, 1008b, and 1009b. The steps 1001b, 1002b, 1003b, 1004b, 1005b, 1006b, and 1007b can be performed similarly to the steps 1001a, 1002a, 1003a, 1004a, 1005a, 1006a, and 1007a respectively. In the step 1008b, the nitrogen and/or hydrogen (H₂) contained in the cooled second reformed gas stream can be reacted to produce ammonia. The step 1008b, can be performed in the unit 400 of the system 100b. In the step 1009b, ammonia and high pressure compressed carbon dioxide can be reacted to form urea. The step 1009b, can be performed in the unit 500 of the system 100b.

The primary reformer 210, of the system 100a, b, can be any suitable primary reformer capable of reacting hydrocarbons and steam used in ammonia production plants. Non limiting examples of reformers can be used as primary reformer include steam reformer, fired tubular reformer, tubular reformer, heat exchange reformers, convection reformers, bayonet reformer, or the like.

The secondary reformer 220, of the system 100a, b, can be any suitable secondary reformer capable of reacting hydrocarbons and air used in ammonia production plants. Non limiting examples of reformers can be used as secondary reformer include adiabatic oxidative reactor, autothermal reactor or the like.

The gas heated reformer 230, of the system 100a, b, can be any suitable reformer capable of reacting hydrocarbons and steam. Non limiting examples of reformers can be used as gas heated reformer include steam reformer, tubular reformer, heat exchange reformers, convection reformers, bayonet reformer, or the like.

The first boiler 240, of the system 100a, b, can be any suitable boiler capable of cooling a gas stream to create steam, used in ammonia production plants. Non limiting examples of steam drum can be used include synthesis loop gas boilers or the like.

The steam drum 250, of the system 100a, b, can be any suitable steam drum capable of separating water from steam used in ammonia production plants. Non limiting examples of steam drum can be used include watertube boiler steam drum or the like.

The second boiler 600, of the system 100b, can be any suitable boiler capable of supplying steam.

The third boiler 700, of the system 100b, can be any suitable boiler capable of receiving steam from ammonia production plants and providing steam to urea production plants. Non limiting examples of boilers can be used as third boilers include outside battery limit (OSBL) boiler.

The first stream 202 and the second stream 208, of the system 100a, b, can contain hydrocarbons and steam. Steam to carbon mole ratio in the first stream 202 and the second stream 208, independently can be 2.7:1 to 4:1, 2.7:1, 2.8:1, 2.9:1, 3:1, 3.1:1, 3.2:1, 3.3:1, 3.4:1, 3.5:1, 3.6:1, 3.7:1, 3.8:1, 3.9:1, or 4:1 or range or value therebetween. In certain embodiments, the first stream 202 and the second stream 208, of the system 100a, b, independently contains at a dry basis i) 78 to 98 mol. %, 78 mol. %, 80 mol. %, 82 mol.%, 84 mol. %, 86 mol. %, 88 mol. %, 90 mol. %, 92 mol.%, 94 mol. %, 96 mol. %, or 98 mol. %, or any value or range therebetween of methane; ii) 0 to 12 mol. %, 0 mol. %, 0.01 mol. %, 0.1 mol.%, 0.5 mol. %, 1 mol. %, 2 mol. %, 4 mol. %, 6 mol. %, 8 mol.%, 10 mol. %, or 12 mol. %, or any value or range therebetween of ethane; iii) 0 to 8 mol. %, 0 mol. %, 0.01 mol. %, 0.1 mol.%, 0.5 mol. %, 1 mol. %, 2 mol. %, 4 mol. %, 6 mol. %, or 8 mol.%, or any value or range therebetween of propane; iv) 0 to 4 mol. %, 0 mol. %, 0.01 mol. %, 0.1 mol.%, 0.5 mol. %, 1 mol. %, 2 mol. %, 3 mol. %, or 4 mol. %, or any value or range therebetween of iso-butane; v) 0 to 4 mol. %, 0 mol. %, 0.01 mol. %, 0.1 mol.%, 0.5 mol. %, 1 mol. %, 2 mol. %, 3 mol. %, or 4 mol. %, or any value or range therebetween of n-butane; and/or vi) 0 to 2 mol. %, 0 mol. %, 0.01 mol. %, 0.1 mol.%, 0.5 mol. %, 1 mol. %, 1.5 mol. %, or 3 mol. %, or any value or range therebetween of C5+ hydrocarbons. The at least a portion of the hydrocarbons in the first stream and steam can be reacted in the primary reformer 210, of the system 100a, b, at 28 to 50 barg, 28 barg, 30 barg, 32 barg, 34 barg, 36 barg, 38 barg, 40 barg, 42 barg, 44 barg, 46 barg, 48 barg, or 50 barg or any range or value therebetween pressure, and/or 680 to 840 °C, 680 °C, 700 °C, 720 °C, 740 °C, 760 °C, 780 °C, 800 °C, 820 °C, or 840 °C, or any range or value therebetween of temperature, in presence of a nickel containing catalyst. In certain embodiments, the first reformed gas stream 205, of the system 100a, b, contains at a dry basis i) 8 to 13 mol.%, 8 mol. %, 9 mol. %, 10 mol.%, 11 mol. %, 12 mol. %, or 13 mol. %, or any value or range therebetween of CO₂; ii) 4 to 12 mol.%, 4 mol. %, 5 mol. %, 6 mol.%, 7 mol. %, 8 mol. %, 9 mol. %, 10 mol.%, 11 mol. %, or 12 mol. %, or any value or range therebetween of CO; iii) 50 to 70 mol. %, 50 mol. %, 52 mol.%, 54 mol. %, 56 mol. %, 58 mol. %, 60 mol. %, 62 mol.%, 64 mol. %, 66 mol. %, 68 mol. %, or 70 mol. %, any value or range therebetween of H₂, and/or iv) 7 to 30 mol. %, 7 mol. %, 8 mol.%, 10 mol. %, 12 mol. %, 14 mol. %, 16 mol. %, 18 mol.%, 20 mol. %, 22 mol. %, 24 mol. %, 26 mol. %, 28 mol. %, or 30 mol. %, any value or range therebetween of methane.

At least a portion of the hydrocarbons in the first reformed gas stream and air can be reacted in the secondary reformer 220, of the system 100a, b, at 27 to 49 barg, 27 barg, 28 barg, 30 barg, 32 barg, 34 barg, 36 barg, 38 barg, 40 barg, 42 barg, 44 barg, 46 barg, 48 barg, or 49 barg or any range or value therebetween of pressure, and/or 900 to 1150 °C, 900 °C, 920 °C, 940 °C, 960 °C, 980 °C, 1000 °C, 1020 °C, 1040 °C, 1060 °C, 1080 °C, 1100 °C, 1120 °C, 1150 °C or 1150 °C, or any range or value therebetween of temperature in presence of nickel containing catalyst. In certain embodiments, the nitrogen gas enriched stream 207, of the system 100a, b, contains at a dry basis i) 6 to 10 mol.%, 6 mol. %, 7 mol. %, 8 mol.%, 9 mol. %, or 10 mol. %, or any value or range therebetween of CO₂; ii) 8 to 14 mol.%, 8 mol. %, 9 mol. %, 10 mol.%, 11 mol. %, 12 mol. %, 13 mol. %, or 14 mol. %, or any value or range therebetween of CO; iii) 45 to 58 mol. %, 45 mol. %, 46 mol. %, 48 mol. %, 50 mol. %, 52 mol.%, 54 mol. %, 56 mol. %, or 58 mol. %, any value or range therebetween of H₂; iv) 20 to 30 mol. %, 20 mol. %, 22 mol. %, 24 mol. %, 26 mol. %, 28 mol. %, or 30 mol. %, any value or range therebetween of N₂; and/or v) 0.2 to 1.8 mol. %, 0.2 mol. %, 0.4 mol.%, 0.6 mol. %, 0.8 mol. %, 1 mol. %, 1.2 mol. %, 1.4 mol.%, 1.6 mol. %, or 1.8 mol. %, any value or range therebetween of methane.

At least a portion of the hydrocarbons in the second stream and at least a portion of the hydrocarbons in the nitrogen gas enriched stream can be reacted with steam in the gas heated reformer 230, of the system 100a, b, at 27 to 49 barg, 27 barg, 28 barg, 30 barg, 32 barg, 34 barg, 36 barg, 38 barg, 40 barg, 42 barg, 44 barg, 46 barg, 48 barg, or 49 barg or any range or value therebetween of pressure, and/or 750 to 850 °C, 750 °C, 760 °C, 770 °C, 780 °C, 790 °C, 800 °C, 810 °C, 820 °C, 830 °C, 850 °C, or 850 °C, or any range or value therebetween of temperature, in presence of a nickel containing catalyst. In certain embodiments, the second reformed gas stream 209, of the system 100a, b, contains at a dry basis i) 6 to 10 mol.%, 6 mol. %, 7 mol. %, 8 mol.%, 9 mol. %, or 10 mol. %, or any value or range therebetween of CO₂; ii) 8 to 14 mol.%, 8 mol. %, 9 mol. %, 10 mol.%, 11 mol. %, 12 mol. %, 13 mol. %, or 14 mol. %, or any value or range therebetween of CO; iii) 45 to 58 mol. %, 45 mol. %, 46 mol. %, 48 mol. %, 50 mol. %, 52 mol.%, 54 mol. %, 56 mol. %, or 58 mol. %, any value or range therebetween of H₂, iv) 20 to 30 mol. %, 20 mol. %, 22 mol. %, 24 mol. %, 26 mol. %, 28 mol. %, or 30 mol. %, any value or range therebetween of N₂; and/or v) 0.2 to 3 mol. %, 0.2 mol. %, 0.4 mol.%, 0.6 mol. %, 0.8 mol. %, 1 mol. %, 1.2 mol. %, 1.4 mol.%, 1.6 mol. %, 1.8 mol. %, or 2 mol. % any value or range therebetween of methane. In the system 100a, b the composition of the cooled second reformed gas stream 263 can be the same as the second reformed gas stream 209.

The hydrocarbon stream 203, of the system 100a, b, can contain i) 78 to 98 mol. %, 78 mol. %, 80 mol. %, 82 mol.%, 84 mol. %, 86 mol. %, 88 mol. %, 90 mol. %, 92 mol.%, 94 mol. %, 96 mol. %, or 98 mol. %, or any value or range therebetween of methane; ii) 0 to 12 mol. %, 0 mol. %, 0.01 mol. %, 0.1 mol.%, 0.5 mol. %, 1 mol. %, 2 mol. %, 4 mol. %, 6 mol. %, 8 mol.%, 10 mol. %, or 12 mol. %, or any value or range therebetween of ethane; iii) 0 to 8 mol. %, 0 mol. %, 0.01 mol. %, 0.1 mol.%, 0.5 mol. %, 1 mol. %, 2 mol. %, 4 mol. %, 6 mol. %, or 8 mol.%, or any value or range therebetween of propane; iv) 0 to 4 mol. %, 0 mol. %, 0.01 mol. %, 0.1 mol.%, 0.5 mol. %, 1 mol. %, 2 mol. %, 3 mol. %, or 4 mol. %, or any value or range therebetween of iso-butane; v) 0 to 4 mol. %, 0 mol. %, 0.01 mol. %, 0.1 mol.%, 0.5 mol. %, 1 mol. %, 2 mol. %, 3 mol. %, or 4 mol. %, or any value or range therebetween of n-butane; and/or vi) 0 to 2 mol. %, 0 mol. %, 0.01 mol. %, 0.1 mol.%, 0.5 mol. %, 1 mol. %, 1.5 mol. %, or 3 mol. %, or any value or range therebetween of C5+ hydrocarbons.

The high pressure compressed carbon dioxide produced by the high pressure electric compressor 320, of the system 100a, b, can have a pressure of > 5 to 40 megapascals, 6 to 35 megapascals, 8 to 30 megapascals, or 10 to 25 megapascals. In certain embodiments, the high pressure compressed carbon dioxide produced by the high pressure electric compressor 320, of the system 100a, b, has a pressure of > 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 megapascals, or any range or value therebetween.

The low pressure carbon dioxide compressed by the steam turbine carbon dioxide compressor 310, of the system 100a, b, can have a pressure of 1 to 5 megapascals. In certain embodiments, the low pressure carbon dioxide compressed by the steam turbine carbon dioxide compressor 310, of the system 100a, b, has a pressure of 0.5, 1, 2, 3, 4, or 5 megapascals, or any range or value therebetween.

In the system 100a, b, the second reformed gas stream 209 produced in the gas heated reformer 230 can have a temperature greater than the cooled second reformed gas stream 263 produced in the first boiler 240. In certain embodiments, the second reformed gas stream 209, of the system 100a, b, produced in the gas heated reformer has a temperature of 700 to 900 °C, 725 to 875 °C, 750 to 850 °C, 775 to 825 °C, 700 °C, 720 °C, 740 °C, 760 °C, 780 °C, 800 °C, 820 °C, 840 °C, 860 °C, 880 °C, or 900 °C, or any value or range therebetween. In certain embodiments, the cooled second reformed stream 263, of the system 100a, b, produced in the first boiler 240 has a temperature of 250 to 500 °C, 225 to 475 °C, 225 to 475 °C, 250 to 450 °C, 275 to 425 °C, 300 to 400 °C, 250 °C, 260 °C, 280 °C, 300 °C, 320 °C, 340 °C, 360 °C, 380 °C, 400 °C, 420 °C, 440 °C, 460 °C, 480 °C, or 500 °C, or any range or value therebetween. In the system 100a, b, the nitrogen gas enriched stream 207 can have a temperature greater than the second reformed gas stream 209 produced in the gas heated reformer 230. In certain embodiments, the nitrogen gas enriched stream 207, of the system 100a, b, has a temperature of 900 °C to 1200 °C, 925 to 1175 °C, 950 to 1150 °C, 975 to 1125 °C, 975 to 1050 °C, 900 °C, 920 °C, 940 °C, 960 °C, 980 °C, 1000 °C, 1020 °C, 1040 °C, 1060 °C, 1080 °C, 1100 °C, 1120 °C, 1140 °C, 1160 °C, 1180 °C, or 1200 °C, or any range or value therebetween. In certain embodiments, the first reformed gas stream 205, of the system 100a, b, produced in the primary reformer has a temperature of 700 to 900 °C, 725 to 875 °C, 750 to 850 °C, 775 to 825 °C, 700 °C, 720 °C, 740 °C, 760 °C, 780 °C, 800 °C, 820 °C, 840 °C, 860 °C, 880 °C, or 900 °C, or any value or range therebetween. In the system 100a, b, the first boiler produced steam can have a temperature higher than that of the water and/or steam that is heated in the first boiler.

In an another embodiment, the steam feed stream 204 is not contacted with the hydrocarbon stream 203 (not shown), and the first stream and second stream is not formed. A first hydrocarbon stream formed from the hydrocarbon steam 203 is the fed to the primary reformer 210 through the first stream and/or steam inlet 211. A second hydrocarbon stream formed from the hydrocarbon steam 203 is the fed to the gas heated reformer 230 through the second stream and/or steam inlet 231. The hydrocarbon stream, the first hydrocarbon stream and the second hydrocarbon stream can have the same composition. A first steam feed stream formed from the steam feed stream 204 can be fed to the primary reformer 210 through an optional steam inlet of the primary reformer. A second steam feed stream formed from the steam feed stream 204 can be fed to the gas heated reformer 230 through an optional steam inlet of the gas heated reformer. In the primary reformer 210 at least a portion of the hydrocarbons in the first hydrocarbon steam can be reacted with steam. The first reformed gas stream can contain the products of the reaction of the at least a portion of the hydrocarbons in the first hydrocarbon steam and steam, and unreacted hydrocarbons from the first hydrocarbon stream. In the gas heated reformer 230 at least a portion of the hydrocarbons in the second hydrocarbon steam, and the at least a portion of the hydrocarbons in the nitrogen gas enriched stream can be reacted with steam. The second reformed gas stream can contain the products of the reaction of the at least a portion of the hydrocarbons in the second hydrocarbon steam and the at least a portion of the hydrocarbons in the nitrogen gas enriched stream with steam, unreacted hydrocarbons from the second hydrocarbon stream, and unreacted hydrocarbons and nitrogen from the nitrogen gas enriched stream

Although embodiments of the present application and their advantages have been described in detail, it should be understood that various changes, substitutions and alterations can be made herein without departing from the spirit and scope of the embodiments as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the above disclosure, processes, machines, manufacture, compositions of matter, means, methods, or steps, presently existing or later to be developed that perform substantially the same function or achieve substantially the same result as the corresponding embodiments described herein can be utilized. Accordingly, the appended claims are intended to include within their scope such processes, machines, manufacture, compositions of matter, means, methods, or steps.

### EXAMPLE

Use of the systems and methods disclosed herein is expected to reduce the steam export to the carbon dioxide compressor by approximately 50 metric tons per day (MTPD) for a 2200 MTPD ammonia and 3850 MTPD urea plant and reduction in the carbon footprint of 120,000 to 180,000 metric tons per annum (MPTA) as compared to using a carbon dioxide compressor that does not use an electric motor. This reduction in the steam exported can allow for use of a primary reformer that is approximately 20% smaller as compared to using a primary reformer without a gas heated reformer and using a carbon dioxide compressor that does not use an electric motor.

## Claims

1. A process for reducing carbon dioxide in production of ammonia and urea, the process comprising:
producing a first reformed gas stream by reacting hydrocarbons in a first stream with steam in a primary reformer;
producing a nitrogen gas enriched stream by reacting hydrocarbons in the first reformed gas stream with air by contacting the first reformed gas stream from the primary reformer and the air with a catalyst in a secondary reformer;
producing a second reformed gas stream by reacting hydrocarbons in the nitrogen gas enriched stream and hydrocarbons in a second stream with steam in a gas heated reformer;
reducing heat of the second reformed gas stream and producing a first boiler produced steam for supporting production of urea by heating water and/or steam in a first boiler with heat from the second reformed gas stream;
compressing carbon dioxide to produce a low pressure compressed carbon dioxide for the production of urea by powering a steam turbine carbon dioxide compressor by the first boiler produced steam; and
compressing the low pressure compressed carbon dioxide to produce a high pressure compressed carbon dioxide by an electric motor driven high pressure electric compressor,
wherein less carbon dioxide is produced than if no gas heated reformer and no electric motor driven high pressure compressor is used, and/or wherein less steam is used to produce the high pressure compressed carbon dioxide than if no electric motor driven high pressure compressor is used.

2. The process of claim 1, wherein the steam reacted in the primary reformer and/or the steam reacted in the gas heated reformer is supplied at least in part by a second boiler.

3. The process of claim 1, wherein the steam reacted in the primary reformer and/or the steam reacted in the gas heated reformer is not supplied by a second boiler.

4. The process of any one of claims 1 to 3, wherein the first stream and the second stream are at least in part supplied by a same hydrocarbon stream.

5. The process of any one of claims 1 to 4, wherein the electric motor is powered by electricity from a renewable source.

6. The process of any one of claims 1 to 5, further comprising reacting the high pressure compressed carbon dioxide with ammonia to produce urea.

7. The process of claim 6, wherein at least a portion of the ammonia is formed by reacting nitrogen from the second reformed gas stream.

8. The process of any one of claims 1 to 7, wherein the high pressure compressed carbon dioxide compressed by the electric motor driven high pressure electric compressor has a pressure of 10 to 25 megapascals.

9. The process of any one of claims 1 to 8, wherein the low pressure compressed carbon dioxide compressed by the steam turbine carbon dioxide compressor has a pressure of 1 to 5 megapascals.

10. A system for production of nitrogen gas and compressed carbon dioxide, the system comprising:
a primary reformer having a steam and/or first stream inlet and a first reformed gas stream outlet;
a secondary reformer having first reformed gas stream inlet in fluid communication with the first reformed gas stream outlet of the primary reformer, the secondary reformer also having an air inlet and a nitrogen gas enriched stream outlet;
a gas heated reformer having a nitrogen gas enriched stream inlet in fluid communication with the nitrogen gas enriched stream outlet of the secondary reformer, the gas heated reformer also having a steam and/or second stream inlet, and a second reformed gas stream outlet;
a first boiler having a second reformed gas stream inlet in fluid communication with the second reformed gas stream outlet of the gas heated reformer, the first boiler also having a water and/or steam inlet, a first boiler produced steam outlet, and a cooled second reformed gas stream outlet;
a steam turbine carbon dioxide compressor having a first boiler produced steam inlet in fluid communication with the first boiler produced steam outlet of the first boiler, the steam turbine carbon dioxide compressor also having a carbon dioxide inlet and a low pressure compressed carbon dioxide outlet;
a high pressure electric compressor having a low pressure compressed carbon dioxide inlet in fluid communication with the low pressure compressed carbon dioxide outlet of the steam turbine carbon dioxide compressor, the high pressure electric compressor also having a high pressure compressed carbon dioxide outlet;
an electric motor in mechanical communication with the high pressure electric compressor.

11. The system of claim 10, further comprising a second boiler having a second boiler steam outlet in fluid communication with the steam and/or first stream inlet of the primary reformer.

12. The system of claim 11, where the second boiler steam outlet is in further fluid communication with the steam and/or second stream inlet of the gas heated reformer.

13. The system of any one of claims 10 to 12, further comprising a renewable energy source in electrical communication with the electric motor.

14. The system of any one of claims 10 to 13, further comprising an ammonia reactor having a cooled second reformed gas stream inlet in fluid communication with the cooled second reformed gas stream outlet of the first boiler, the ammonia reactor is configured to react nitrogen and hydrogen to produce ammonia.

15. The system of any one of claims 10 to 14, further comprising a urea reactor having a high pressure compressed carbon dioxide inlet in fluid communication with the high pressure compressed carbon dioxide outlet of the high pressure electric compressor and the urea reactor having an ammonia inlet, preferably the ammonia inlet is in fluid communication with the ammonia reactor in fluid communication with the first boiler.
